# EUROPÄISCHE PATENTSCHRIFT

(11) **EP 2 510 977 B1**
(45) Veröffentlichungstag und Bekanntmachung des Hinweises auf die Patenterteilung: **02.04.2014**
(21) Anmeldenummer: 12155721.9
(22) Anmeldetag: 16.02.2012
(51) Int. Cl.: A61N 5/10

(54) **Verfahren und Vorrichtung zur Bestrahlungsplanung**
Method and device for irradiation planning
Procédé et dispositif destinés à la planification d'irradiation

(30) Priorität: 15.04.2011 DE 102011007498; 31.05.2011 DE 102011076771
(43) Veröffentlichungstag der Anmeldung: 17.10.2012
(73) Patentinhaber: Siemens Aktiengesellschaft, 80333 München (DE)
(72) Erfinder: Chen, Wenjing, 91058 Erlangen (DE); Gemmel, Alexander, 91054 Erlangen (DE); Rietzel, Eike, 64331 Weiterstadt (DE)

(56) Entgegenhaltungen:
- WO-A2-2010/018476
- PAUL KEALL: "4-Dimensional Computed Tomography Imaging and Treatment Planning", SEMINARS IN RADIATION ONCOLOGY, SAUNDERS, PHILADELPHIA, PA, US, Bd. 14, Nr. 1, 1. Januar 2004 (2004-01-01) , Seiten 81-90, XP002461023, ISSN: 1053-4296, DOI: 10.1053/J.SEMRADONC.2003.10.006

## Beschreibung

Die Erfindung betrifft ein Verfahren und eine Vorrichtung Bestrahlungsplanung sowie eine Bestrahlungsanlage mit einer derartigen Vorrichtung. Eine derartige Vorrichtung bzw. ein derartiges Verfahren findet üblicherweise Einsatz im Rahmen einer Strahlentherapie, insbesondere im Rahmen einer Partikeltherapie.

Im Vorfeld einer Strahlentherapie ist es notwendig, einen Bestrahlungsplan zu bestimmen. Diese Bestimmung ist mitunter schwierig, da sich die interne Anatomie eines Patienten mit der Zeit verändern kann. Beispielsweise können Zielvolumina innerhalb des Abdomens ihre Lage von Tag zu Tag ändern oder im Verlauf mehrerer Tage oder Wochen. Ein typisches Organ, das oftmals einer Positionsänderung unterliegt, ist die Prostata. So können z.B. die neben der Prostata gelegene Blase und das neben der Prostata gelegene Rektum einen Einfluss auf die Lage und die Form der Prostata haben, je nach Füllungszustand der Blase und/oder des Rektums.

Eine Möglichkeit, diese Veränderungen zu berücksichtigen, ist die Verwendung von Sicherheitssäumen. Bei der Bestrahlungsplanung werden derartige Sicherheitssäume so gewählt, dass eine interne Verschiebung/Verformung des Zielvolumens berücksichtigt wird. Obwohl mit diesen Sicherheitssäumen die negativen Auswirkungen einer Lageänderung des Zielvolumens gemildert werden können, können diese Sicherheitssäume zu einer Bestrahlung von benachbarten, kritischen Strukturen führen, wie beispielsweise der Blase oder des Rektums.

Aus der Schrift US 2005/0201516 A1 ist das Konzept bekannt, für ein Zielvolumen mehreren Bestrahlungspläne mit unterschiedlichen Sicherheitssäumen zu berechnen (MMODS für engl.: "multiple-margin optimization with daily selection"). Ein Anwender kann dann in Echtzeit aus einer Vielzahl von optimierten Bestrahlungsplänen mit unterschiedlichen Sicherheitssäumen denjenigen auswählen, um eine beobachtete Veränderung in der Größe oder Position des Tumors oder den Tumor umgebenden Strukturen zu berücksichtigen.

Die US 2010/0088339 A1 offenbart ein Verfahren, bei der aus einer Vielzahl von zuvor erstellten Bestrahlungsplänen vor einer Bestrahlungsfraktion der am besten passende Bestrahlungsplan ausgewählt wird.

Es ist die Aufgabe der Erfindung, ein Verfahren bzw. eine Vorrichtung zur Bestrahlungsplanung bereitzustellen, der es erlaubt, einen Bestrahlungsplan bereitzustellen, der mögliche Lage- oder Formveränderungen des Zielvolumens berücksichtigt. Weiterhin ist es die Aufgabe der Erfindung, eine Bestrahlungsanlage mit einer derartigen Vorrichtung anzugeben. Weiterhin ist es die Aufgabe der Erfindung, eine Bestrahlungsanlage mit einer derartigen Vorrichtung anzugeben.

Die Aufgabe der Erfindung wird gelöst durch die Gegenstände der unabhängigen Ansprüche.

Bei dem Verfahren zur Bestrahlungsplanung für ein zu bestrahlendes Zielvolumen werden folgende Schritte ausgeführt.

Zunächst wird eine Mehrzahl von Datensätzen vorgegeben, in jeweils denen das zu bestrahlendes Zielvolumen abgebildet ist. Die Datensätze unterschieden sich dadurch, dass das Zielvolumen eine andere Position und/oder Form hat. Beispielsweise können die Datensätze 3D-Abbildungsdatensätze sein, wie z.B. Computertomographien.

Aus diesen Datensätzen wird ein Datensatz ausgewählt. Die anderen Datensätze werden dann zu diesem ausgewählten Datensatz registriert. Die Registrierungen, z.B. rigide, lineare oder affine Registrierungen, werden dazu verwendet, die Datensätze hinsichtlich ihrer Position und/oder Skalierung aneinander anzugleichen. Die Registrierungen können derart sein, dass die transformierten Zielvolumina nicht vollständig zueinander transformiert werden, sodass sie dann deckungsgleich wären, sondern behalten ihre unterschiedliche Charakteristik bzgl. Form- und/oder Position bei. Nach Registrierung und Transformation sind die Datensätze und damit die Zielvolumina aber direkt miteinander vergleichbar. Die Registrierung erhält also die während die aufgrund einer unterschiedlichen Patientenanatomie bedingten Unterschiede zwischen den Datensätzen zumindest teilweise.

Anschließend wird eine Vereinigung der in den Datensätzen mit unterschiedlicher Position und/oder Form vorliegenden Zielvolumina gebildet, und zwar unter Verwendung der Registrierungen der anderen Datensätze zu dem ausgewählten Datensatz.

In dem ausgewählten Datensatz wird zusätzlich ein Risikobereich bestimmt, der insbesondere bei einer Bestrahlung geschützt werden soll. Dies können z.B. ein oder mehrere Risikoorgane sein oder - allgemeiner - ein oder mehrere Volumina kritischer Strukturen.

Die Vereinigung der Zielvolumina wird anschließend modifiziert, indem der Risikobereich von der Vereinigung der Zielvolumina wieder ausgenommen, also entfernt wird.

Diese modifizierte Vereinigung der Zielvolumina wird bei einer Berechnung des Bestrahlungsplans herangezogen. Die modifizierte Vereinigung der Zielvolumina kann dabei das zu bestrahlende Volumen kennzeichnen, das mit einer Solldosis zu bestrahlen ist.

Die Datensätze, beispielsweise Abbildungsdatensätze eines Patienten wie Computertomographien, bilden das zu bestrahlende Zielvolumen und üblicherweise ein oder mehrere Volumina kritischer Strukturen (zum Beispiel von so genannten Risikoorganen, auch "organs at risk" - OAR genannt) ab.

Das oder die Zielvolumina und das oder die Volumina kritischer Strukturen können bereits gekennzeichnet sein bzw. im den Datensätzen bereits konturiert vorliegen. Dies kann beispielsweise geschehen, indem die Datensätze mithilfe eines automatischen oder halbautomatischen Segmentierungsverfahrens segmentiert werden. Die Kennzeichnung kann aber auch vollständig manuell durchgeführt werden. Letztlich bedeutet die Kennzeichnung, dass eine Zuordnung bestimmter Bereiche des Datensatzes zu dem Zielvolumen und/oder zu einer kritischen Struktur vorliegt, dass bestimmte Bereiche des Datensatzes als Zielvolumen und/oder als Volumen einer kritischen Struktur markiert sind.

Bei dem Verfahren berücksichtigt der so berechnete Bestrahlungsplan folglich mögliche Lage- bzw. Positionsänderungen des Zielvolumens (indem er mehrere Datensätze und tatsächlich, in Realität vorkommende Lageveränderungen des Zielvolumens berücksichtigt und eine Vereinigung verschiedener Zielvoluminapositionen bzw. -formen bildet), berücksichtigt aber die spezifische Position der kritischen Strukturen (indem die kritische Struktur nur desjenigen Datensatzes entfernt wird, der als Ausgangspunkt des Verfahrens ausgewählt wurde). Auf diese Weise wird trotz der Erweiterung des Zielvolumens sichergestellt, dass keine kritischen Strukturen getroffen werden, wie sie an einem spezifischen Tag vorliegen, und dass nur das Notwendige von der Vereinigung der Zielvolumina entfernt wird.

Das Verfahren kann mehrfach wiederholt angewendet werden. Bei jeder Wiederholung wird ein anderer Datensatz aus der Mehrzahl von Datensätzen als ausgewählter Datensatz gewählt. Damit lässt sich bei jeder Wiederholung ein anderer Bestrahlungsplan erhalten, wobei jeder dieser Bestrahlungspläne dann dem Datensatz zugeordnet ist, der bei Beginn des Verfahrens als ausgewählter Datensatz gewählt wurde. Auf diese Weise steht eine Mehrzahl von Bestrahlungsplänen zur Verfügung, welche ganz spezifisch jeweils die Lage von Risikobereichen berücksichtigt.

Die Datensätze können bevorzugter Weise an unterschiedlichen Tagen aufgezeichnet werden. Auf diese Weise ist die Wahrscheinlichkeit höher, dass in den einzelnen Datensätzen auch eine geänderte Patientenanatomie vorliegt und dass damit mögliche Lageänderungen in adäquater Weise berücksichtigt werden.

So kann z.B. einer oder mehrere der Datensätze ein Verifikationsdatensatz sein, der bei einer Positionierung eines Patienten mit dem Zielvolumen in einem Bestrahlungsraum verwendet wird. Der Verifikationsdatensatz kann z.B. am Behandlungstag im Vorfeld einer Behandlungssitzung aufgezeichnet werden. Anhand des Verifikationsdatensatzes kann der Patient entsprechend positioniert werden, so dass im Zielvolumen an einem Ort liegt, an dem eine passgenaue Bestrahlung möglich wird. Ob der Patient dann anschließend tatsächlich in dieser Position bestrahlt wird, hängt von einem Anwender ab, beispielsweise einem Arzt, der im Einzelfall entscheidet, ob die Bestrahlung dann tatsächlich durchgeführt wird oder ob die Bestrahlung zum Beispiel aufgrund der körperlichen Verfassung des Patienten nicht stattfinden soll.

Dieses Verfahren hat den Vorteil, dass Verifikationsdatensätze, die ohnehin meistens zu Positionierung des Patienten aufgezeichnet werden, gleichzeitig dafür verwendet werden können, Bestrahlungspläne nach den oben geschilderten Verfahren zu erstellen.

Eine Fortbildung führt zu einem Verfahren zum Bestimmen eines Bestrahlungsplans für ein zu bestrahlendes Zielvolumen.

In einem ersten Schritt wird dabei ein Verfahren nach einem der Ansprüche 2 bis 4 ausgeführt, sodass den Datensätzen der Mehrzahl von Datensätzen jeweils ein Bestrahlungsplan zugeordnet ist. Jedem Datensatz ist dabei derjenige Bestrahlungsplan zugeordnet, der mit einer modifizierten Vereinigung an Zielvolumina erstellt wurde, bei der von der Vereinigung der Zielvolumina ein Risikobereich entfernt wurde, und zwar mit seiner spezifischen Position bzw. Lage, wie er in dem jeweiligen Datensatz vorgelegen hat.

In einem zweiten, dem ersten Schritt nachfolgenden Schritt werden folgende Schritte ausgeführt:
i) Aufzeichnen eines Verifikationsdatensatzes,
ii) Vergleichen des Verifikationsdatensatzes mit denjenigen Datensätzen, denen ein Bestrahlungsplan zugeordnet ist, zur Bestimmen desjenigen Datensatzes, der eine größte Ähnlichkeit mit dem Verifikationsdatensatz aufweist, und
iii) Auswählen desjenigen Bestrahlungsplans, der diesem Datensatz zugeordnet ist.

Auf diese Weise kann sichergestellt werden, dass für eine mögliche Bestrahlung derjenige Bestrahlungsplan ausgewählt wird, der am besten der aktuellen Patientenanatomie entspricht. Dieser Bestrahlungsplan kann dann der Bestrahlungseinrichtung übermittelt werden und zum Beispiel in deren Speicher geladen werden, damit, falls ein Arzt die Bestrahlung freigibt, dieser Bestrahlungsplan dann von der Bestrahlungseinrichtung appliziert werden kann.

In einer Ausführungsform wird für den Vergleich in Schritt ii) ein Bereich in den Datensätzen bestimmt. Der Vergleich wird lediglich in diesem Bereich durchgeführt. Auf diese Weise kann sichergestellt werden, dass nur relevante Bildbereiche für die Beurteilung der Patientenanatomie zum Vergleich herangezogen werden. Unterschiede in Bereichen, die beispielsweise weit entfernt von dem zu bestrahlenden Zielvolumen liegen und die daher keinen nennenswerten Einfluss auf die Relevanz bei der Auswahl der passenden Bestrahlungspläne haben, können so bei dem Vergleich ausgeklammert werden.

So kann der Bereich ein an das das Zielvolumen angrenzendes Organ nur teilweise umfassen, z.B. nur diejenigen Seite des Organs, die dem Zielvolumen zugewandt ist und die daher im sogenannten "Interface"-Bereich zum Zielvolumen liegt. Weiterhin können bestimmte Gebiete aus dem Bereich, indem der Vergleich der Datensätze stattfindet, ausgeklammert werden. So können beispielsweise Gebiete, die durch Lufteinschlüsse charakterisiert sind, von dem Bereich nicht umfasst sein und gezielt ausgeklammert werden. Dasselbe gilt auch für Gebiete, die durch bestimmte, vordefinierte Gewebearten charakterisiert sind, wie beispielsweise Knochengewebe.

Dies alles schränkt den Bereich für den Vergleich auf Gebiete ein, die sich für die Auswahl eines passenden Bestrahlungsplans als besonders relevant herausgestellt haben.

Die Vorrichtung zur Bestrahlungsplanung für ein zu bestrahlendes Zielvolumen, weist eine Rechnereinheit auf, welche - beispielsweise durch Ausbildung entsprechender Module - ausgebildet bzw. konfiguriert ist:
- zum Laden einer Mehrzahl von Datensätzen, in jeweils denen das zu bestrahlendes Zielvolumen abgebildet ist, wobei sich die Datensätze dadurch unterschieden, dass das Zielvolumen eine andere Position und/oder Form aufweist,
- zum Auswählen eines Datensatzes aus der Mehrzahl der Datensätze
- zum Registrieren der anderen Datensätze zu dem ausgewählten Datensatz, und
- zum Bilden einer Vereinigung der Zielvolumina unter Verwendung der Registrierungen der anderen Datensätze zu dem ausgewählten Datensatz,
- zum Bestimmen eines Risikobereichs in dem ausgewählten Datensatz,
- zum Modifizieren der Vereinigung der Zielvolumina, indem der Risikobereich von der Vereinigung der Zielvolumina ausgenommen wird, und
- zum Berechnen eines Bestrahlungsplans unter Verwendung des modifizierten Zielvolumens.

Die Rechnereinheit der Bestrahlungsplanungsvorrichtung kann zur Durchführung eines Verfahrens nach einem der Ansprüche 1 oder 2 ausgebildet bzw. konfiguriert sein.

Die Rechnereinheit kann weiterhin ausgebildet bzw. konfiguriert sein zum Vergleichen eines Verifikationsdatensatzes mit den Datensätzen der Mehrzahl der Datensätze. Insbesondere kann die Rechnereinheit ausgebildet bzw. konfiguriert sein zum Bestimmen eines Bereichs für den Vergleich des Verifikationsdatensatzes mit den Datensätzen der Mehrzahl der Datensätze, und zur Durchführung des Vergleichs lediglich in diesem Bereich.

Die vorangehende und die folgende Beschreibung der einzelnen Merkmale, deren Vorteile und deren Wirkungen bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Ausführungsformen der Erfindung werden anhand der folgenden Zeichnung näher erläutert, ohne jedoch darauf beschränkt zu sein. Es zeigen:
- Fig. 1: fünf verschiedene Abbildungsdatensätze, in denen jeweils die Prostata, die Blase und das Rektum eingezeichnet sind,
- Fig. 2: eine schematische Darstellung der Auswahl eines Datensatzes und die Registrierung der anderen Datensätze auf diesen Datensatz,
- Fig. 3: eine schematische Darstellung eines Abbildungsdatensatzes, in dem das Zielvolumen eingezeichnet ist sowie die Position von zwei Risikoorganen und die entsprechende Lage von Zielvolumina aus anderen Abbildungsdatensätzen,
- Fig. 4: eine schematische Darstellung der Maske, die zum Vergleich der Ähnlichkeit bei zwei Datensätzen herangezogen wird,
- Fig. 5: ein Ablaufdiagramm einer Ausführungsform des Verfahrens, und
- Fig. 6: eine Bestrahlungsplanungsvorrichtung, mit der eines der offenbarten Verfahren ausgeführt werden kann.

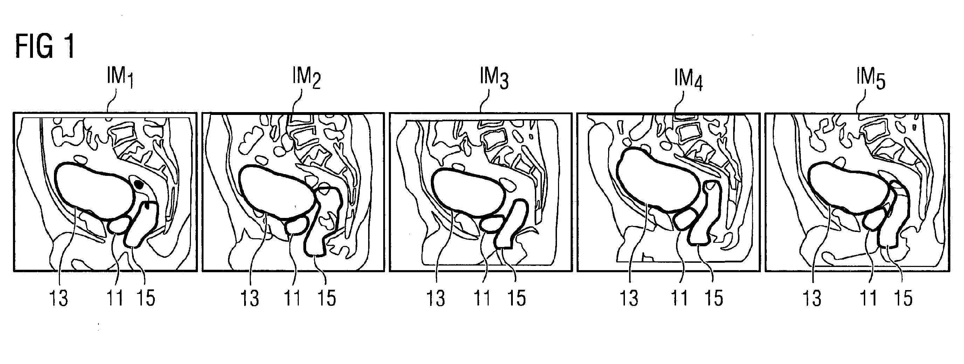

Fig. 1 zeigt den Ausgangspunkt des Verfahrens zur Bestrahlungsplanung, nämlich mehrere (hier: fünf) unterschiedliche Datensätze, die dieselbe zu bestrahlende Anatomie abbilden. Da die Datensätze zu unterschiedlichen Zeitpunkten angefertigt wurden, ist die Anatomie des Patienten leicht geändert. Die unterschiedlichen n Datensätze werden mit IM1, IM2, ... bis IMn (hier: IM5) bezeichnet.

In Fig. 1 zu sehen sind jeweils Datensätze IM1 ... IM5, in denen das zu bestrahlende Zielvolumen (hier: die Prostata 11 des Patienten) und zu schonende Risikoorgane (hier: die Blase 13 und das angrenzende Rektum 15) bereits segmentiert vorliegen und entsprechend eingezeichnet sind. Aufgrund des unterschiedlichen Füllungszustandes der Blase 13 und des Rektums 15 sind die Position und die Form des Zielvolumens und die Volumina der kritischen Strukturen von Datensatz zu Datensatz leicht anders.

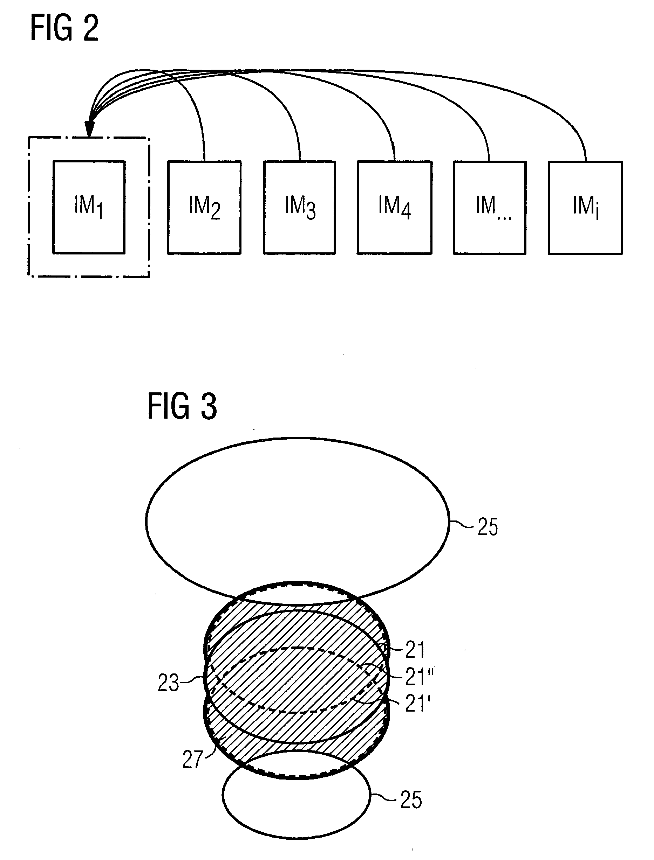

In einem ersten Schritt werden alle Datensätze bis auf den ersten Datensatz, also IM2 ... IMn, zu dem ersten Datensatz IM 1 registriert, wie in Fig. 2 skizziert angedeutet.

Diese Registrierungen werden verwendet, um die Datensätze miteinander vergleichbar zu machen. Die Registrierungen gleichen zwar der Datensätze hinsichtlich Translation, Rotation und Skalierung aneinander an, sodass die Datensätze direkt miteinander vergleichbar werden. Die Registrierungen erhalten jedoch - zumindest teilweise - die spezifischen Lage- und Positionsunterschiede des Zielvolumens und des oder der Volumina der kritischen Strukturen, die aufgrund der unterschiedlichen Patientenanatomie zum Zeitpunkt der Aufnahme der Datensätze bedingt sind.

Die Registrierungen werden verwendet, um die konturierten Zielvolumina 11 der anderen Datensätze auf den ersten Datensatz entsprechend zu transformieren.

Fig. 3 verdeutlicht nun, wie mit den ggf. transformierten Zielvolumina verfahren wird.

Anschließend werden alle Zielvolumina, also das Zielvolumen 21 des ersten Datensatzes IM1 und die auf den ersten Datensatz transformierten Zielvolumina 21, 21" der weiteren Datensätze IM2 ... IMn vereint. Hierdurch wird ein Vereinigungs-Zielvolumen 23 gebildet.

Von diesem Vereinigungs-Zielvolumen 23 werden wiederum das oder die Volumina 25 der kritischen Strukturen entfernt, wie sie im ersten Datensatz IM1 vorliegen.

Dies führt zu einem ersten globalen Zielvolumen, im Folgenden als (GP-OAR)1 bezeichnet (grau hinterlegter Bereich 27).

Dieses Verfahren wird für Indices i = 1 ... n durchgeführt. Dies bedeutet, dass beim i-ten Index:
- die Datensätze IM1 ... IMi-1, IMi+1, IMn zu dem i-ten Datensatz IMi registriert werden,
- die Zielvolumina in den Datensätzen IM1 ... IMi-1, IMi+1, IMn anhand der Registrierungen auf den i-ten Datensatz IMi transformiert werden,
- ein Vereinigungs-Zielvolumen aus dem IMi-Zielvolumen und den transformierten Zielvolumina gebildet wird, und anschließend von diesem Vereinigungs-Zielvolumen das oder die Volumina der kritischen Strukturen, wie es im i-ten Datensatz IMi vorliegt, wieder entfernt werden,
   sodass dann ein i-tes globales Zielvolumen erhalten wird, als (GP-OAR)i bezeichnet.

Anschließend wird für jeden Index i = 1 ... n ein Bestrahlungsplan TPi erstellt. Diese Bestrahlungsplanung verwendet den Datensatz IMi und das nach dem obigen Verfahren bestimmte i-te globale Zielvolumen (GP-OAR)i und kann - basierend auf diesen Vorgaben - nach herkömmlichen Bestrahlungsplanungsverfahren durchgeführt werden.

So können bevorzugter Weise das oder die Volumina der zu schonenden Strukturen, die in dem Datensatz IMi vorliegen, derart berücksichtigt werden, dass für diese Volumina einzuhaltende Randbedingungen festgelegt werden, wie z.B. eine maximal zu applizierende Dosis oder eine Dosis-Volumen-Randbedingung.

Die Mehrzahl von Bestrahlungsplänen TPi, i = 1 ... n wird dann in einer Bestrahlungsplan-Datenbank hinterlegt. Jeder Bestrahlungsplan TPi ist dabei dem Ausgangs-Datensatz IMi, auf den die anderen Datensätze registriert wurden, zugeordnet.

Dies alles findet in einem ersten Schritt statt, z.B. im Vorfeld einer geplanten Bestrahlungssitzung.

Bevor nun die Bestrahlungssitzung begonnen wird, wird ein zusätzlicher Datensatz IMd aufgezeichnet, der die aktuelle Position des Zielvolumens und damit die aktuelle Patientengeometrie abbildet, und der im Folgenden als Verifikationdatensatz bezeichnet wird. Dieser Verifikationsdatensatz kann z.B. dann dazu verwendet werden, den Patienten in einem Behandlungsraum oder für eine geplante Behandlung zu positionieren. Dadurch kann das Zielvolumen an passendem Ort positioniert werden, damit das Zielvolumen wie geplant bestrahlt werden kann.

Der Verifikationsdatensatz IMd wird dazu verwendet, den geeigneten Bestrahlungsplan aus der Mehrzahl der Bestrahlungspläne TPi auszuwählen.

Hierzu findet ein Vergleich des Verifikationsdatensatzes IMd mit den Datensätzen IMi statt, um ein Ähnlichkeitsmaß zu ermitteln, das angibt, wie ähnlich der Verifikationsdatensatz IMd zu den Datensätzen IMi ist. Hierzu können verschiedene bekannte Verfahren verwendet werden, wie z.B. Summe der quadratischen Differenzen (engl: "sum of squared differences"), Korrelationskoeffizienten (engl: "correlation coefficient"), oder gegenseitige Information (engl: "mutual information"), um die Ähnlichkeit mit einem Maß zu quantifizieren.

Bevorzugterweise wird die Ähnlichkeit nicht für die gesamte vorliegende Bildinformation der Datensätze verglichen, sondern nur in einem bestimmten Bildbereich, einem bestimmten Sub-Volumen, auch als Maske bezeichnet.

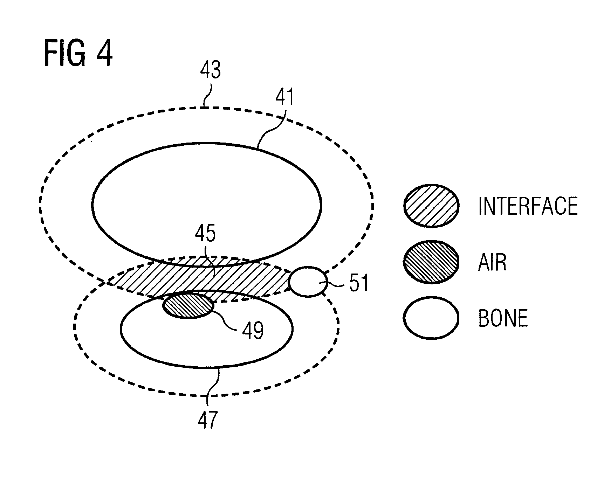

Anhand von Fig. 4 wird nun erläutert, wie so eine Maske bestimmt werden kann.

Die Maske kann wie folgt ermittelt werden: Im Datensatz IMi wird das Zielvolumen 41 zunächst mit Hilfe einer Box zu einem erweiterten Zielvolumen 43 erweitert.

Z.B. kann der Datensatz ein Computertomographie-Datensatz (CT-Datensatz) sein mit einer Schichtdicke von 3 mm (z-Richtung) und einer Pixelgröße von 2x2 mm (x- bzw. y-Richtung). Bei einer Box-Größe von 3x3x2 Voxel (in x-, y- ,z-Richtung) ergibt sich eine geometrische Box-Größe von 6 mm x 6 mm x 6 mm. Falls der CT-Datensatz eine andere Auflösung hat, kann die Größe der Erweiterungs-Box entsprechend angepasst werden, um eine gewünschte geometrische Größe der Erweiterungs-Box zu erreichen.

Anschließend wird das erweiterte Zielvolumen mit den Übergangsstellen 45 (engl.: "interface") zu angrenzenden, zu schonenden Organen 47 (auch als OAR für "Organs At Risk" bezeichnet) erweitert. Im Falle einer Prostata als Zielvolumen können die Übergangsstellen zur Blase und zum Rektum gewählt werden.

Die Übergangsstellen können ermittelt werden, indem die überlappenden Voxel zwischen Zielvolumen 41 und OAR 47 genommen werden, die anschließend erweitert zu einem erweiterten Übergangsbereich werden.

Beispielsweise kann bei dem oben erwähnten CT-Datensatz-Beispiel für die Erweiterung der Übergangsstellen eine Erweiterungs-Box mit einer Größe von 5x5x3 Voxel (in x-, y-, z-Richtung) genommen werden. Bei der Erweiterung der Übergangsstellen können Voxel ausgenommen werden, die Luft (engl: "air") 49 enthalten oder die einem bestimmten, vordefinierten Gewebe, wie z.B. Knochen (engl: "bone") 51, zugeordnet werden können.

Die Maske kann dann gebildet werden, indem das erweiterte Zielvolumen mit dem erweiterten Übergangsbereich vereint wird.

Die Maske kennzeichnet den Bereich des i-ten Datensatzes IMi, welcher für den Vergleich mit dem aktuellen Verifikationsdatensatz herangezogen wird und welcher hinsichtlich der Ähnlichkeit bewertet wird.

Anschließend kann der Bestrahlungsplan TPi gewählt werden, der dem Datensatz IMi zugeordnet ist, der die höchste gegenseitige Information, den höchsten Korrelationskoeffizienten und/oder die geringste Summe der quadratischen Abweichungen beim Vergleich mit dem Verifikationsdatensatz IMd aufweist.

Der Bestrahlungsplan TPi kann dann exportiert werden und/oder in ein Bestrahlungssystem geladen werden. Dadurch steht der Bestrahlungsplan für eine evtl. stattfindende nachfolgende Bestrahlung zur Verfügung.

In der klinischen Praxis ist es nicht üblich, im Vorfeld einer Bestrahlung mehrere CT-Datensätze aufzuzeichnen, um daraus mehrer Bestrahlungspläne zu berechnen.

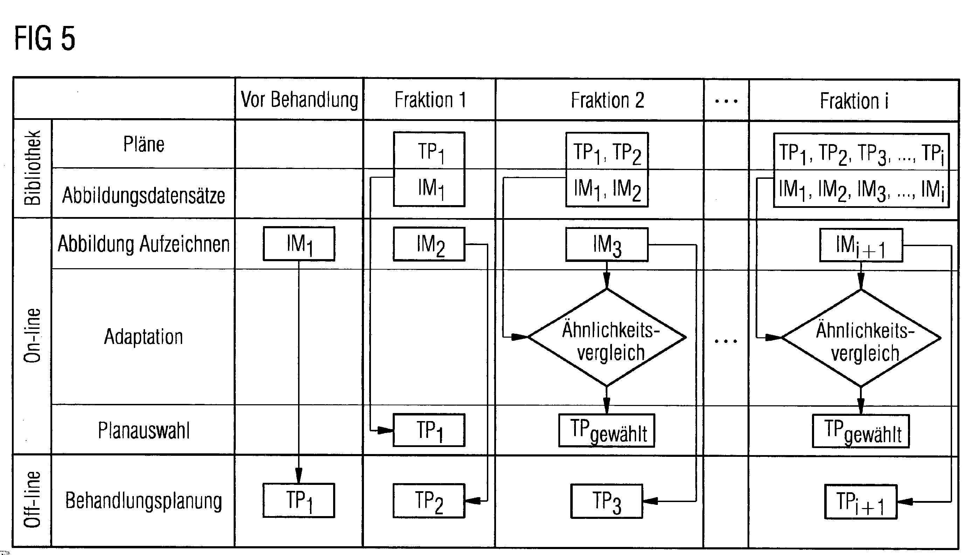

Das oben vorgestellte Verfahren kann aber derart implementiert werden, dass eine Datenbank mit möglichen Bestrahlungsplänen TPi sukzessive erweitert wird. Das vorgeschlagene Ablaufdiagramm wird anhand von Fig. 5 erläutert.

Im Vorfeld einer geplanten Bestrahlung wird ein erster CT-Datensatz IM1 erstellt und die Planung beruht auf diesem Datensatz IM1 (linke Spalte "Vor Behandlung"). Hierdurch wird ein erster Bestrahlungsplan TP1 erstellt.

Bei einer ersten Bestrahlungssitzung (Spalte "Fraktion 1") kann der erste Bestrahlungsplan TP1 ausgewählt und in das Bestrahlungssystem geladen werden. Falls der Arzt oder Anwender seine Zustimmung gibt, kann der ersten Bestrahlungsplan TP1 appliziert werden.

Im Rahmen der ersten Bestrahlungssitzung wird ein weiterer CT-Datensatz IM2 erstellt, der bei der ersten Bestrahlungssitzung für die Positionierung des Patienten bzw. zur Positionsverifikation verwendet wird.

Im Anschluss an die erste Bestrahlungssitzung liegen nun zwei Datensätze vor, IM1 und IM2. Das anhand von Fig. 1 bis Fig. 4 beschriebene Verfahren kann dann "offline" angewandt werden, um aus IM2 unter Einbeziehung von IM1 einen zweiten Bestrahlungsplan TP2 zu ermitteln.

Für die zweite Bestrahlungssitzung (Spalte "Fraktion 2") liegen dann zwei Bestrahlungspläne TP1 und TP2 vor. Ein zur Positionierung des Patienten aufgezeichneter Verifikationsdatensatz IM3 für die zweite Bestrahlungssitzung kann dann einerseits zur Positionsverifikation verwendet werden. Andererseits kann dieser Verifikationsdatensatz IM3 mit den Datensätzen IM1 und IM2 verglichen werden, um über das oben beschriebene Verfahren zur Ermittlung des Ähnlichkeitsmaßes den am besten passenden Bestrahlungsplan aus den beiden vorliegenden Bestrahlungsplänen TP1 und TP2 zu ermitteln.

Das Verfahren kann bei weiteren Fraktionen weiter in analoger Weise ausgeführt werden (z.B. Spalte "Fraktion i") :
Für eine i-te Bestrahlungssitzung wird ein Verifikationsdatensatz IMi+1 aufgezeichnet, der dann mit den bereits vorliegenden Datensätzen IM1 ... IMi verglichen wird, um den besten aus den bereits erstellten Bestrahlungsplänen TP1 ... TPi auszuwählen. Offline kann dann der Verifikationsdatensatz IMi+1 als Datensatz IMi+1 dienen, um die Bibliothek der vorliegenden Bestrahlungspläne TP1 ... TPi um einen weiteren Bestrahlungsplan TPi+1 zu erweitern.

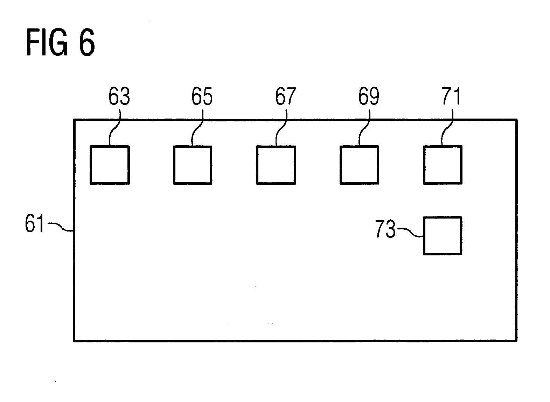

Fig. 6 zeigt die Komponenten einer Bestrahlungsplanungsvorrichtung, mit der die oben geschilderten Verfahren ausgeführt werden können. Die Vorrichtung und deren Komponenten können beispielsweise in einem Rechnersystem 61 realisiert werden.

Die Vorrichtung zur Bestrahlungsplanung umfasst eine Schnittstelle 63, über die Datensätze geladen werden können.

Hierüber können eine Mehrzahl von Datensätzen, in jeweils denen das zu bestrahlendes Zielvolumen abgebildet ist, wobei sich die Datensätze dadurch unterschieden, dass das Zielvolumen eine andere Position und/oder Form aufweist, geladen werden.

Die Vorrichtung umfasst eine Auswahleinrichtung 65, die derart konfiguriert ist, dass ein Datensatz aus einer Mehrzahl ausgewählt wird.

Die Vorrichtung umfasst eine Registrierungseinrichtung 67, die derart konfiguriert ist, dass - basierend auf der Auswahl der Auswahleinrichtung - andere Datensätze zu dem ausgewählten Datensatz registriert werden.

Die Vorrichtung umfasst ferner eine Zielvolumen-Bestimmungs-Einrichtung 69, die derart konfiguriert ist, dass eine Vereinigung der Zielvolumina unter Verwendung der Registrierungen der anderen Datensätze zu dem ausgewählten Datensatz gebildet wird. Ferner ist die Zielvolumen-Bestimmungs-Einrichtung konfiguriert zum Modifizieren der Vereinigung der Zielvolumina, indem der Risikobereich von der Vereinigung der Zielvolumina ausgenommen wird.

Die Vorrichtung umfasst eine Planungseinrichtung 71, die konfiguriert ist, unter Verwendung der modifizierten Zielvolumen-Vereinigung basierend auf den Soll-Vorgaben eine Bestrahlungsplanung durchzuführen.

Die Vorrichtung weist zudem eine Vergleichseinrichtung 73 auf, die konfiguriert ist zum Vergleichen eines Verifikationsdatensatzes mit den Datensätzen der Mehrzahl der Datensätze. Die Vergleichseinrichtung ist insbesondere derart konfiguriert, dass ein Bereich für den Vergleich des Verifikationsdatensatzes mit den Datensätzen der Mehrzahl der Datensätze bestimmt wird, und dass der Vergleich lediglich in diesem Bereich durchgeführt wird.

Die Arbeiten, die zu dieser Erfindung geführt haben, wurden gemäß der Finanzhilfevereinbarung Nr. 215840 im Zuge des Siebten Rahmenprogramms der Europäischen Union (RP7/2007-2013) gefördert.

## Patentansprüche

1. Verfahren zur Bestrahlungsplanung für ein zu bestrahlendes Zielvolumen (21, 21', 21"), umfassend folgende Schritte:
a) Vorgeben einer Mehrzahl von Datensätzen (IMi ... IMn), in jeweils denen das zu bestrahlendes Zielvolumen abgebildet ist, wobei sich die Datensätze (IM1 ... IMn) dadurch unterschieden, dass das Zielvolumen (21, 21', 21") eine andere Position und/oder Form aufweist,
b) Wählen eines Datensatzes aus der Mehrzahl der Datensätze (IM1 ... IMn) und Registrieren anderer Datensätze aus der Mehrzahl der Datensätze (IM1 ... IMn) zu dem ausgewählten Datensatz,
c) Bilden einer Vereinigung (23) der Zielvolumina (21, 21', 21") unter Verwendung der Registrierungen der anderen Datensätze zu dem ausgewählten Datensatz,
d) Bestimmen eines Risikobereichs (25) in dem ausgewählten Datensatz,
e) Modifizieren der Vereinigung der Zielvolumina (21, 21', 21"), indem der Risikobereich (25) von der Vereinigung (23) der Zielvolumina (21, 21', 21") ausgenommen wird,
f) Berechnen eines Bestrahlungsplans (TP1 ... TPn) unter Verwendung der modifizierten Vereinigung (27) der Zielvolumina (21, 21', 21") .

2. Verfahren nach Anspruch 1,
wobei eine Mehrzahl von Bestrahlungsplänen (TP1 ... TPn) berechnet wird, indem die Schritte b) bis f) wiederholt werden, wobei bei jeder Wiederholung ein anderer Datensatz aus der Mehrzahl von Datensätzen (IM1 ... IMn) als ausgewählter Datensatz gewählt wird.

3. Verfahren nach Anspruch 1 oder 2,
wobei die Datensätze (IM1 ... IMn) an unterschiedlichen Tagen aufgezeichnet werden.

4. Verfahren nach einem der Ansprüche 1 bis 3,
wobei zumindest ein Datensatz der Mehrzahl der Datensätze (IM1 ... IMn) ein Verfikationsdatensatz (IMd) ist, der bei einer Positionierung eines Patienten mit dem Zielvolumen (21, 21', 21") für eine geplante Behandlung erwendet wird.

5. Verfahren zum Bestimmen eines Bestrahlungsplans für ein zu bestrahlendes Zielvolumen (21, 21', 21"), umfassend folgende Schritte:
- in einem ersten Schritt: Ausführen eines Verfahrens nach einem der Ansprüche 2 bis 4 ausgeführt, sodass den Datensätzen der Mehrzahl von Datensätzen (IM1 ... IMn) jeweils ein Bestrahlungsplan (TP1 ... TPn) zugeordnet ist,
- in einem zweiten, dem ersten Schritt nachfolgenden Schritt:
i) Aufzeichnen eines Verifikationsdatensatzes (IMd),
ii) Vergleichen des Verifikationsdatensatzes (IMd) mit denjenigen Datensätzen (IM1 ... IMn), denen ein Bestrahlungsplan (TP1 ... TPn) zugeordnet ist, zur Bestimmen desjenigen Datensatzes, der eine größte Ähnlichkeit mit dem Verifikationsdatensatz aufweist, und
iii) Auswählen desjenigen Bestrahlungsplans, der diesem Datensatz zugeordnet ist.

6. Verfahren nach Anspruch 5,
wobei für den Vergleich in Schritt ii) ein Bereich in den Datensätzen bestimmt wird, und der Vergleich lediglich in diesem Bereich durchgeführt wird.

7. Verfahren nach Anspruch 6, wobei der Bereich ein an das das Zielvolumen (41) angrenzendes Organ (47) nur teilweise umfasst.

8. Verfahren nach Anspruch 6 oder 7, wobei der Bereich ein Gebiet, das durch Luft (49) oder durch ein bestimmtes Gewebe (51) charakterisiert ist, nicht umfasst.

9. Vorrichtung zur Bestrahlungsplanung für ein zu bestrahlendes Zielvolumen (21, 21', 21"), aufweisend:
ein Rechnersystem (61), welche ausgebildet ist:
- zum Laden einer Mehrzahl von Datensätzen (IM1 ... IMn), in jeweils denen das zu bestrahlendes Zielvolumen abgebildet ist, wobei sich die Datensätze (IM1 ... IMn) dadurch unterschieden, dass das Zielvolumen (21, 21', 21") eine andere Position und/oder Form aufweist,
- zum Auswählen eines Datensatzes aus der Mehrzahl der Datensätze (IM1 ... IMn),
- zum Registrieren der anderen Datensätze zu dem ausgewählten Datensatz, und
- zum Bilden einer Vereinigung (23) der Zielvolumina unter Verwendung der Registrierungen der anderen Datensätze zu dem ausgewählten Datensatz,
- zum Bestimmen eines Risikobereichs (25) in dem ausgewählten Datensatz,
- zum Modifizieren der Vereinigung (23) der Zielvolumina, indem der Risikobereich (25) von der Vereinigung der Zielvolumina ausgenommen wird, und
- zum Berechnen eines Bestrahlungsplans unter Verwendung des modifizierten Zielvolumens (27).

10. Vorrichtung zur Bestrahlungsplanung nach Anspruch 9, wobei das Rechnersystem (61) ausgebildet zur Durchführung eines Verfahrens nach einem der Ansprüche 1 oder 2.

11. Vorrichtung zur Bestrahlungsplanung nach einem der Ansprüche 9 oder 10,
wobei das Rechnersystem (61) weiterhin ausgebildet ist zum Vergleichen eines Verifikationsdatensatzes (IMd) mit den Datensätzen der Mehrzahl der Datensätze (IM1 ... IMn).

12. Vorrichtung zur Bestrahlungsplanung nach Anspruch 10 oder 11, wobei das Rechnersystem (61) ausgebildet ist zum Bestimmen eines Bereichs für den Vergleich des Verifikationsdatensatzes (IMd) mit den Datensätzen der Mehrzahl der Datensätze (IM1 ... IMn), und zur Durchführung des Vergleichs lediglich in diesem Bereich.

13. Vorrichtung zur Bestrahlungsplanung nach Anspruch 12, wobei
das Rechnersystem (61) ausgebildet ist zum Bestimmen des Bereichs, indem der Bereich ein an das das Zielvolumen (41) angrenzendes Organ (47) nur teilweise umfasst.

14. Vorrichtung zur Bestrahlungsplanung nach Anspruch 12 oder 13, wobei der Bereich ein Gebiet, das durch Luft (49) oder durch ein bestimmtes Gewebe (51) charakterisiert ist, nicht umfasst.

## Claims

1. Method for irradiation treatment planning for a target volume to be irradiated (21, 21', 21"), comprising the following steps:
a) specification of a plurality of data sets (IMi ... IMn), in each of which the target volume to be irradiated is depicted, wherein the data sets (IM1 ... IMn) differ in that the target volume (21, 21', 21") has another position and/or shape,
b) selection of a data set from the plurality of the data sets (IM1 ... IMn) and registration of other data sets from the plurality of the data sets (IM1 ... IMn) to the selected data set,
c) formation of an amalgamation (23) of the target volumes (21, 21', 21") using the registrations of the other data sets to the selected data set,
d) determination of an area at risk (25) in the selected data set,
e) modification of the amalgamation of the target volumes (21, 21', 21") in that the area at risk (25) is excluded from the amalgamation (23) of the target volumes (21, 21', 21"),
f) calculation of an irradiation treatment plan (TP1 ... TPn) using the modified amalgamation (27) of the target volumes (21, 21', 21").

2. Method according to claim 1,
wherein a plurality of irradiation treatment plans (TP1 ... TPn) is calculated in that steps b) to f) are repeated, wherein on each repetition another data set from the plurality of data sets (IM1 ... IMn) is selected as the selected data set.

3. Method according to claim 1 or 2,
wherein the data sets (IM1 ... IMn) are recorded on different days.

4. Method according to any one of claims 1 to 3,
wherein at least one data set of the plurality of data sets (IM1 ... IMn) is a verification data set (IMd), which is used for planned treatment during the positioning of a patient with the target volume (21, 21', 21").

5. Method for the determination of an irradiation treatment plan for a target volume to be irradiated (21, 21', 21"), comprising the following steps:
- in a first step: execution of a method according to any one of claims 2 to 4 so that each of the data sets of the plurality of data sets (IM1 ... IMn) is assigned an irradiation treatment plan (TP1 ... TPn),
- in a second step following the first step:
i) recording of a verification data set (IMd),
ii) comparison of the verification data set (IMd) with the data sets (IM1 ... IMn) to which an irradiation treatment plan (TP1 ... TPn) is assigned for the determination of the data set with the greatest similarity to the verification data set and
iii) selection of the irradiation treatment plan assigned to this data set.

6. Method according to claim 5,
wherein, for the comparison in step ii), an area in the data sets is determined and the comparison is only performed in this area.

7. Method according to claim 6, wherein the area only partially includes an organ (47) adjacent to the target volume (41).

8. Method according to claim 6 or 7, wherein the area does not include a region **characterised by** air (49) or by a specific tissue (51).

9. Device for irradiation treatment planning for a target volume to be irradiated (21, 21', 21"), comprising:
a computer system (61), which is designed:
- to load a plurality of data sets (IM1 ... IMn), in each of which the target volume to be irradiated is depicted, wherein the data sets (IM1 ... IMn) differ in that the target volume (21, 21', 21") has another position and/or shape,
- for the selection of a data set from the plurality of the data sets (IM1 ... IMn),
- for the registration of the other data sets to the selected data set, and
- for the formation of an amalgamation (23) of the target volumes using the registrations of the other data sets to the selected data set,
- for the determination of an area at risk (25) in the selected data set,
- for the modification of the amalgamation (23) of the target volumes in that the area at risk (25) is excluded from the amalgamation of the target volumes and
- for the calculation of an irradiation treatment plan using the modified target volume (27).

10. Device for irradiation treatment planning according to claim 9,
wherein the computer system (61) is designed for the execution of a method according to any one of claims 1 or 2.

11. Device for irradiation treatment planning according to either claim 9 or claim 10,
wherein the computer system (61) is also designed for the comparison of a verification data set (IMd) with the data sets of the plurality of the data sets (IM1 ... IMn).

12. Device for irradiation treatment planning according to either claim 10 or claim 11, wherein the computer system (61) is designed for the determination of an area for the comparison of the verification data set (IMd) with the data sets of the plurality of the data sets (IM1 ... IMn) and for the performance of the comparison in this area only.

13. Device for irradiation treatment planning according to claim 12, wherein
the computer system (61) is designed for the determination of the area in that the area only partially includes an organ (47) adjacent to the target volume (41).

14. Device for irradiation treatment planning according to claim 12 or claim 13, wherein the area does not include a region **characterised by** air (49) or by a specific tissue (51).

## Revendications

1. Procédé destiné à la planification d'irradiation pour un volume cible à irradier (21, 21', 21"), comprenant les étapes suivantes :
a) détermination d'une pluralité de jeu de données (IMi...
IMn), le volume cible à irradier étant reproduit dans chacun d'entre eux, les jeux de données (IMi... IMn) se différenciant par le fait que le volume cible (21, 21', 21") présente une autre position et/ou forme,
b) sélection d'un jeu de données à partir d'une pluralité de jeux de données (IMi... IMn) et enregistrement des autres jeux de données issus de la pluralité de jeux de données (IMi... IMn) dans le jeu de données sélectionné,
c) formation d'une union (23) des volumes cibles (21, 21', 21") en utilisant les enregistrements des autres jeux de données dans le jeu de données sélectionné,
d) détermination d'une zone de risque (25) dans le jeu de données sélectionné,
e) modification de l'union des volumes cibles (21, 21', 21") en extrayant la zone de risque (25) de l'union (23) des volumes cibles (21, 21', 21"),
f) calcul d'un plan d'irradiation (TP1... TPn) en utilisant l'union modifiée (27) des volumes cibles (21, 21', 21").

2. Procédé selon la revendication 1,
dans lequel une pluralité de plans d'irradiation (TP1... TPn) sont calculés, les étapes b) à f) étant répétées, dans lequel lors de chaque répétition un autre jeu de données issu de la pluralité de jeux de données (IMi... IMn) est sélectionné en tant que jeu de données sélectionné.

3. Procédé selon la revendication 1 ou 2,
dans lequel les jeux de données (IMi... IMn) sont enregistrés sur des jours différents.

4. Procédé selon l'une des revendications 1 à 3,
dans lequel au moins un jeu de données de la pluralité de jeux de données (IMi... IMn) est un jeu de données de vérification (IMd), qui est utilisé pour un traitement planifié lors du positionnement d'un patient avec le volume cible (21, 21', 21").

5. Procédé destiné à la détermination d'un plan d'irradiation pour un volume cible à irradier (21, 21', 21"), comprenant les étapes suivantes :
- lors d'une première étape : réalisation d'un procédé selon l'une des revendications 2 à 4, de sorte qu'un plan d'irradiation (TP1... TPn) est à chaque fois associé aux jeux de données de la pluralité de jeux de données (IMi... IMn),
- lors d'une deuxième étape consécutive à la première étape :
i) enregistrement d'un jeu de données de vérification (IMd),
ii) comparaison du jeu de données de vérification (IMd) avec les jeux de données (IMi... IMn) auquel est associé un plan d'irradiation (TP1... TPn), afin de déterminer le jeu de données qui présente la plus grande similitude avec le jeu de données de vérification, et
iii) sélection du plan d'irradiation qui est associé à ce jeu de données.

6. Procédé selon la revendication 5,
dans lequel pour la comparaison à l'étape ii) une zone est déterminée dans le jeu de données, et la comparaison n'est exécutée que dans cette zone.

7. Procédé selon la revendication 6, dans lequel la zone ne comprend que partiellement un organe (47) adjacent au volume cible (41).

8. Procédé selon la revendication 6 ou 7, dans lequel la zone ne comprend pas de région qui est **caractérisée par** l'air (49) ou par un tissu déterminé (51).

9. Dispositif destiné à la planification d'irradiation pour un volume cible à irradier (21, 21', 21"), présentant :
un système informatique (61), qui est configuré :
- pour le chargement d'une pluralité de jeux de données (IMi... IMn), dans chacun desquels est reproduit le volume cible à irradier, les jeux de données (IMi... IMn) se différenciant par le fait que le volume cible (21, 21', 21") présente une autre position et/ou forme,
- pour la sélection d'un jeu de données à partir d'une pluralité de jeux de données (IMi... IMn),
- pour l'enregistrement des autres jeux de données dans le jeu de données sélectionné, et
- pour la formation d'une union (23) des volumes cibles en utilisant les enregistrements des autres jeux de données dans le jeu de données sélectionné,
- pour la détermination d'une zone de risque (25) dans le jeu de données sélectionné,
- pour la modification de l'union (23) des volumes cibles en extrayant la zone de risque (25) de l'union des volumes cibles, et
- pour le calcul d'un plan d'irradiation en utilisant le volume cible modifié (27).

10. Dispositif destiné à la planification d'irradiation selon la revendication 9,
dans lequel le système informatique (61) est configuré pour la réalisation d'un procédé selon l'une des revendications 1 ou 2.

11. Dispositif destiné à la planification d'irradiation selon l'une des revendications 9 ou 10,
dans lequel le système informatique (61) est en outre configuré pour la comparaison d'un jeu de données de vérification (IMd) avec le jeux de données de la pluralité de jeux de données (IMi... IMn).

12. Dispositif destiné à la planification d'irradiation selon la revendication 10 ou 11, dans lequel le système informatique (61) est configuré pour la détermination d'une zone en vue de la comparaison du jeu de données de vérification (IMd) avec les jeux de données de la pluralité de jeux de données (IMi... IMn) et pour la réalisation de la comparaison uniquement dans cette zone.

13. Dispositif destiné à la planification d'irradiation selon la revendication 12, dans lequel
le système informatique (61) est configuré pour la détermination de la zone dans laquelle la zone ne comprend que partiellement un organe (47) adjacent au volume cible (41).

14. Dispositif destiné à la planification d'irradiation selon la revendication 12 ou 13, dans lequel la zone ne comprend pas de région qui est **caractérisée par** l'air (49) ou par un tissu déterminé (51).
